(19)

**Europäisches Patentamt**
**European Patent Office**
**Office européen des brevets**

(11) **EP 4 607 530 A1**

(12) **EUROPEAN PATENT APPLICATION**

(43) Date of publication:
**27.08.2025 Bulletin 2025/35**

(21) Application number: **25159273.9**

(22) Date of filing: **21.02.2025**

(51) International Patent Classification (IPC):
*G16H 30/40* (2018.01)     *A61B 5/00* (2006.01)
*A61B 8/08* (2006.01)     *A61B 8/00* (2006.01)
*G06T 7/00* (2017.01)     *G16H 50/50* (2018.01)
*A61B 5/02* (2006.01)

(52) Cooperative Patent Classification (CPC):
**G16H 50/50; A61B 5/0044; A61B 5/02028;**
**A61B 8/0883; A61B 8/5261; G06T 7/0012;**
**G16H 30/40;** G06T 2207/30048

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **23.02.2024 IN 202421013196**

(71) Applicant: **Tata Consultancy Services Limited**
**Maharashtra (IN)**

(72) Inventors:
 • **KANAKATTE GURUMURTHY, Aparna**
   **560009 Bengaluru, Karnataka (IN)**
 • **GHOSE, Avik**
   **700091 Kolkata, West Bengal (IN)**
 • **BHATIA, Divya Manoharlal**
   **560009 Bangalore, Karnataka (IN)**
 • **MAZUMDER, Oishee**
   **700156 Kolkata, West Bengal (IN)**
 • **SINHA, Aniruddha**
   **700091 Kolkata, West Bengal (IN)**
 • **AGARWAL, Swapna**
   **700156 Kolkata, West Bengal (IN)**
 • **KHANDELWAL, Sundeep**
   **201301 Noida, Uttar Pradesh (IN)**
 • **CHAUDHURY, Rupam**
   **110001 New Delhi, Delhi (IN)**
 • **BANERJEE, Shilajit**
   **700160 Kolkata, West Bengal (IN)**
 • **DAS, Sudip Kumar**
   **700160 Kolkata, West Bengal (IN)**
 • **CHANDEL, Vivek**
   **122004 Gurugram, Haryana (IN)**

(74) Representative: **Goddar, Heinz J.**
   **Boehmert & Boehmert**
   **Anwaltspartnerschaft mbB**
   **Pettenkoferstrasse 22**
   **80336 München (DE)**

(54) **METHOD FOR GENERATING A PERSONALIZED 4D CARDIAC MODEL**

(57)     Planning cardiac surgery is a complex task. Current heart models are mostly static and provide no functional information or personalization apart from structural information from imaging modalities like MRI, CT etc. The embodiments of present disclosure addresses unresolved problem of generating a personalized four dimensional cardiac model that integrates blood flow velocity, pressure profile and volume on three-dimensional cardiac structure, providing a comprehensive tool for pre or post-surgical planning and analysis. Unlike existing techniques, which often rely on separate assessments of cardiac anatomy and hemodynamics, using invasive methods, the present disclosure provides a 4D cardiac model that captures the person specific dynamic interaction between cardiac structures and blood flow throughout the cardiac cycle. Further, the 4D cardiac model enables clinicians to visualize and quantify complex flow patterns and pressure distributions within the region of interest in the heart, facilitating precise surgical planning and outcome prediction especially in pediatric heart surgery.

200

| receiving a plurality of multi-modal images of a heart | 202 |
| generating a 3D cardiac structure and a first reference blood flow velocity from the plurality of multi-modal images using segmentation | 204 |
| selecting a region of interest (ROI) from the 3D cardiac structure | 206 |
| measuring a blood flow velocity profile and a pressure profile throughout the ROI using modified LBM model. | 208 |
| generating a 4D cardiac model by registering the blood flow velocity and the pressure profile with the 3D cardiac structure using optical flow algorithms. | 210 |

FIG 2

**Description**

CROSS-REFERENCE TO RELATED APPLICATIONS AND PRIORITY

The present application claims priority from Indian provisional patent application no. 202421013196, filed on February 23, 2024.

TECHNICAL FIELD

The disclosure herein generally relates to medical imaging and modelling techniques, and, more particularly, to a method and system for generating a personalized four-dimensional (4D) cardiac model by registering hemodynamics over three-dimensional (3D) cardiac structural model.

BACKGROUND

Cardiac surgery and especially pediatric cardiac surgery for congenital cardiac disorders, is a niche domain with unique challenges. The small size of the pediatric heart coupled with the variety and uniqueness of genetic defects, makes surgeries very challenging. These challenges demand a system that can aid the pediatric cardiac surgeons in planning their surgeries more effectively.

Medical imaging plays a crucial role in generating a (three-dimensional) 3D or (four-dimensional) 4D cardiac model and thereby in the diagnosis of cardiac anomalies. A 3D cardiac model is a digital representation of structural details of human heart or a specific region inside the human heart. A 4D model, which includes spatial as well as temporal dimensions, facilitates in observing the changes in cardiac structure and functionalities over a specific period. Current 3D and 4D heart models are mostly static and provide no functional information or personalization to an individual apart from structural information from imaging modalities like computerized tomography (CT), and magnetic resonance imaging (MRI).

There are several imaging modalities like X-ray, echocardiography, single-photon emission computed tomography (SPECT), positron emission tomography (PET), computerized tomography (CT), and magnetic resonance imaging (MRI) that aid in diagnosing cardiac diseases. The heart is a dynamic structure that continuously deforms throughout the cardiac pumping cycle. Consequently, relying on a single imaging modality gives inadequate information for the attending cardiologist. For the purpose of collecting as much information on the cardiac systems as possible towards effective treatment planning, the expert recommends multi-modality images. Some imaging modalities, such as echocardiography and fluoroscopy/angiography, are often used during interventional procedures to provide visual aid to the physician/surgeon. In certain clinical cases, multiple images are acquired at different time points or from different viewpoints and also from different imaging modalities. Thus, image fusion is often useful for the integration of various sources of information. The primary step of this fusion is image registration.

Image registration is a complex process that involves aligning and integrating multiple medical images, acquired at various instants and under various conditions especially using various imaging modalities to fabricate a coherent and synchronized representation of the cardiac system. Conventional approaches toward replicating cardiac functionality are limited to individual functions like hemodynamics or electrophysiology. In hemodynamics domain, zero dimensional (0D) lumped model and 3D computational fluid dynamics model exists concentrating on specific cardiac regions.

The purpose of image registration is to align images with respect to each other to provide complementary information from the integrated information from each modality. For example, echocardiography can provide functional information such as functional information of cardiac valves, valve regurgitation, stenosis, and systolic function and measure systolic ejection performance and the extent of wall hypertrophy. However, the image quality of echocardiography is inferior to other three-dimensional (3D) imaging modalities due to the presence of speckle noise and limited field of view (FOV). The 3D imaging modalities such as CT and MRI can provide quantitative assessments on valve morphology, valvular dysfunction severity, and structural information of chambers and vessels. The major areas where this image registration can be used is to facilitate image segmentation, surgical guidance, in motion correction etc.

Cardiac image registration is a more complex and challenging problem than brain image registration because of the nonrigid and mixed motions of the heart and the thorax structures. The heart exhibits fewer accurate anatomical landmarks and also the cardiac images are usually acquired with a lower resolution than brain images. The basic steps in the image registration algorithm often involve feature extraction, geometrical transformation definition, similarity measure, and optimization.

Current methods of cardiac image registration such as geometric attribute registration, voxel similarity registration, and mutual information (MI) registration are based on the voxel intensities. In inter-modality imaging, appropriate landmarks may not be available in both modalities and voxel intensities are often very dissimilar. This leads to difficulty in implementing landmark and similarity registration methods. MI registration is a method used frequently in inter-modality registration and is a measure of the statistical dependence between two random variables. For multimodality imaging, corresponding pixel intensities are the random variables in MI registration. It has been shown that MI is not capable of accurately registering PET and CT images for attenuation correction. Thus, exploring enhanced image registering techniques is an open area for research.

SUMMARY

Embodiments of the present disclosure present technological improvements as solutions to one or more of the above-mentioned technical problems recognized by the inventors in conventional systems. For example, in one embodiment, a system for generating a personalized four-dimensional (4D) cardiac model is provided. The system includes a memory storing instructions; one or more Input/Output (I/O) interfaces; and one or more hardware processors coupled to the memory via the one or more I/O interfaces, wherein the one or more hardware processors are configured by the instructions to receive a plurality of multi-modal images of a heart, wherein the plurality of multi-modal images includes Magnetic Resonance Imaging (MRI) and echocardiography (ECHO) images. The system is configured to generate a three-dimensional (3D) cardiac structure from the plurality of multi-modal images of the heart. The 3D cardiac structure is generated utilizing a histogram-based image segmentation approach. During the segmentation process the heart region is isolated from surrounding tissues to ensure that subsequent flow analysis is focused solely on the relevant anatomical structures. The extraction of the heart region and generation of the 3D cardiac structure involves the following steps: Initially, the plurality of multi-modal images, for example MRI dataset comprising a series of two-dimensional (2D) slice frames forming 3D volume, is loaded sequentially. Sequential loading ensures continuity within the MRI dataset and preserves spatial relationships between slices, thereby maintaining the 3D anatomical structure of the heart. Subsequently, each 2D slice is converted to a grayscale format to emphasize intensity variations across tissue types. Converting to Grayscale format eliminates color information that is irrelevant for medical imaging, instead focusing on intensity values that correspond to tissues such as myocardium, blood, and fat. Later, a two-dimensional Gaussian Blur filter is applied to each 2D slice to reduce noise and smoothen the images. The filtering step minimizes high-frequency noise that could interfere with accurate segmentation, particularly in detailed images, and enhances the definition of cardiac structures for subsequent processing stages. Then, thresholding is performed on each 2D slice to isolate the heart region based on the intensity values of cardiac tissues. Threshold values are selected to differentiate heart tissues from surrounding structures, wherein the thresholding operation is conducted independently for each 2D slice, ensuring accurate identification of cardiac structures within each frame. Next, a plurality of filtered 2D slices is generated for each 2D slice after thresholding by retaining only the pixels corresponding to the heart region, wherein the filtering removes extraneous tissues, ensuring that only the relevant cardiac tissue is preserved for further analysis. Finally, each among the plurality of filtered 2D slices are combined to generate a 3D mask that isolates the heart region from surrounding tissues across the entire MRI volume. The subtraction of the plurality of filtered 2D slices from the original 3D volume emphasizes the 3D cardiac structure.

Further, the system is configured to select a region of interest (ROI) from the 3D cardiac structure generated after segmentation of the plurality of multi-modal images. The ROI is defined based on the specific area of the heart being studied, such as the ventricles, atria, or heart valves. The ROI can be of any shape and size, allowing flexibility depending on the region under analysis. The objective of selecting the ROI is analyzing the blood flow in certain cardiac regions, such as through a valve or a specific vessel. Further, the system is configured to concurrently estimate a reference blood flow velocity from the 3D cardiac structure, especially within the ROI. The reference blood flow velocity estimation includes the following steps: calculating a set of flow vectors within the ROI using an optical flow algorithm such as Fernback optical flow algorithm. The set of flow vectors are calculated from speed of the blood through the ROI. The optical flow algorithm works by analyzing pixel intensity changes between successive MRI frames (slices) over time. In 3D volumes, analyzing pixel intensity means that for each frame (slice) of the MRI, the optical flow algorithm calculates an optical flow (motion) of the blood from one frame/slice to the next, capturing both spatial and temporal variations. The optical flow over the 3D cardiac structure is then scaled using blood flow information at specific regions (e.g. through Mitral valve, aortic valve etc.) obtained from the plurality of multi-modal images, say a 2D Echo report. This scaled 3D blood flow is termed as reference blood flow. The speed of the blood flow calculated from the above formulae in cm/s is considered as the Reference blood flow velocity.

Further, the system is configured to generate a patient specific reference model by registering the reference blood flow over the 3D cardiac structure. Then, the system is configured for measuring a blood flow velocity profile and a pressure profile throughout the ROI using modified Lattice Boltzmann Method based computation model (LBM model). The blood flow velocity profile is computed by performing a sequence of steps, wherein the sequence of steps include: Initially, computing wall velocity and pressure profile of blood flow within the ROI based on structural analysis of the 3D cardiac structure. The wall velocity is imported from the MRI 3D structural analysis. And, concurrently obtaining a set of boundary conditions for the ROI, wherein the set of boundary conditions includes an inlet velocity and an inlet pressure of the blood flow captured from a lumped zero-dimensional (0D) hemodynamic model. The lumped 0D hemodynamic model is a closed-loop, real-time, cardiovascular simulation model representing dynamics of four cardiac chambers, heart valves, and lumped pulmonary and systemic circulation. Finally, calculating the blood flow velocity profile and pressure profile within the ROI based on the wall velocity, pressure profile and the set of boundary conditions using the modified LBM model.

Further, the system is configured for generating a four-dimensional (4D) cardiac model by registering the blood flow velocity profile and the pressure profile with the 3D cardiac structure using optical flow algorithms. The 4D cardiac model also embeds a volume derived from the blood flow velocity and the pressure profile as its fourth dimension. Finally, the 4D cardiac model is calibrated using the reference model, wherein the blood flow velocity profile is calibrated with the reference blood flow velocity and wherein the calibration is made by adjusting the inlet velocity and inlet pressure captured from the lumped 0D hemodynamics model.

In another aspect, a method for generating a personalized four-dimensional (4D) cardiac model is provided. The method includes: receiving via one or more hardware processors a plurality of multi-modal images of a heart, wherein the plurality of multi-modal images includes Magnetic Resonance Imaging (MRI) and echocardiography (ECHO) images. The method via the one or more hardware processors generate a three-dimensional (3D) cardiac structure from the plurality of multi-modal images of the heart. The 3D cardiac structure is generated utilizing a histogram-based image segmentation approach. During the segmentation process the heart region is isolated from surrounding tissues to ensure that subsequent flow analysis is focused solely on the relevant anatomical structures. The extraction of the heart region and generation of the 3D cardiac structure involves the following steps: Initially, the plurality of multi-modal images, for example MRI dataset comprising a series of two-dimensional (2D) slice frames forming 3D volume, is loaded sequentially. Sequential loading ensures continuity within the MRI dataset and preserves spatial relationships between slices, thereby maintaining the 3D anatomical structure of the heart. Subsequently, each 2D slice is converted to a grayscale format to emphasize intensity variations across tissue types. Converting to Grayscale format eliminates color information that is irrelevant for medical imaging, instead focusing on intensity values that correspond to tissues such as myocardium, blood, and fat. Later, a two-dimensional Gaussian Blur filter is applied to each 2D slice to reduce noise and smoothen the images. The filtering step minimizes high-frequency noise that could interfere with accurate segmentation, particularly in detailed images, and enhances the definition of cardiac structures for subsequent processing stages. Then, thresholding is performed on each 2D slice to isolate the heart region based on the intensity values of cardiac tissues. Threshold values are selected to differentiate heart tissues from surrounding structures, wherein the thresholding operation is conducted independently for each 2D slice, ensuring accurate identification of cardiac structures within each frame. Next, a plurality of filtered 2D slices is generated for each 2D slice after thresholding by retaining only the pixels corresponding to the heart region, wherein the filtering removes extraneous tissues, ensuring that only the relevant cardiac tissue is preserved for further analysis. Finally, each among the plurality of filtered 2D slices are combined to generate a 3D mask that isolates the heart region from surrounding tissues across the entire MRI volume. The subtraction of the plurality of filtered 2D slices from the original 3D volume emphasizes the 3D cardiac structure.

Further, the method via the one or more hardware processors select a region of interest (ROI) from the 3D cardiac structure generated after segmentation of the plurality of multi-modal images. The ROI is defined based on the specific area of the heart being studied, such as the ventricles, atria, or heart valves. The ROI can be of any shape and size, allowing flexibility depending on the region under analysis. The objective of selecting the ROI is analyzing the blood flow in certain cardiac regions, such as through a valve or a specific vessel. Further, the method via the one or more hardware processors is configured to concurrently estimate a reference blood flow velocity from the 3D cardiac structure, especially within the ROI. The reference blood flow velocity estimation includes the following steps: calculating a set of flow vectors within the ROI using an optical flow algorithm such as Fernback optical flow algorithm. The set of flow vectors are calculated from speed of the blood through the ROI. The optical flow algorithm works by analyzing pixel intensity changes between successive MRI frames (slices) over time. In 3D volumes, analyzing pixel intensity means that for each frame (slice) of the MRI, the optical flow algorithm calculates an optical flow (motion) of the blood from one frame/slice to the next, capturing both spatial and temporal variations. The optical flow over the 3D cardiac structure is then scaled using blood flow information at specific regions (e.g. through Mitral valve, aortic valve etc.) obtained from the plurality of multi-modal images, say a 2D Echo report. This scaled 3D blood flow is termed as reference blood flow. The speed of the blood flow calculated from the above formulae in cm/s is considered as the Reference blood flow velocity.

Further, the method via the one or more hardware processors is configured to generate a patient specific reference model by registering the reference blood flow over the 3D cardiac structure. Then, the method via the one or more hardware processors measure a blood flow velocity profile and a pressure profile throughout the ROI using modified Lattice Boltzmann Method based computation model (LBM model). The blood flow velocity profile is computed by performing a sequence of steps, wherein the sequence of steps include: Initially, computing wall velocity and pressure profile of blood flow within the ROI based on structural analysis of the 3D cardiac structure. The wall velocity is imported from the MRI 3D structural analysis. And, concurrently obtaining a set of boundary conditions for the ROI, wherein the set of boundary conditions includes an inlet velocity, and an inlet pressure of the blood flow captured from a lumped zero-dimensional (0D) hemodynamic model. The lumped 0D hemodynamic model is a closed-loop, real-time, cardiovascular simulation model representing dynamics of four cardiac chambers, heart valves, and lumped pulmonary and systemic circulation. Finally, calculating the blood flow velocity profile and pressure profile within the ROI based on the wall velocity, pressure profile and the set of boundary conditions using the modified LBM model.

Further, the method via the one or more hardware processors is configured to generate a four-dimensional (4D) cardiac model by registering the blood flow velocity profile and the pressure profile with the 3D cardiac structure using optical flow algorithms. The 4D cardiac model also embeds a volume derived from the blood flow velocity and the pressure profile as its fourth dimension. Finally, the 4D cardiac model is calibrated using the reference model, wherein the blood flow velocity profile is calibrated with the reference blood flow velocity and wherein the calibration is made by adjusting the inlet velocity and inlet pressure captured from the lumped 0D hemodynamics model.

In yet another aspect, a non-transitory computer readable medium comprising one or more instructions, which when executed by one or more hardware processors causes a method for generating a personalized four-dimensional (4D) cardiac model, is provided. The method includes: receiving via one or more hardware processors a plurality of multi-modal images of a heart, wherein the plurality of multi-modal images includes Magnetic Resonance Imaging (MRI) and echocardiography (ECHO) images. The method via the one or more hardware processors generate a three-dimensional (3D) cardiac structure from the plurality of multi-modal images of the heart. The 3D cardiac structure is generated utilizing a histogram-based image segmentation approach. During the segmentation process the heart region is isolated from surrounding tissues to ensure that subsequent flow analysis is focused solely on the relevant anatomical structures. The extraction of the heart region and generation of the 3D cardiac structure involves the following steps: Initially, the plurality of multi-modal images, for example MRI dataset comprising a series of two-dimensional (2D) slice frames forming 3D volume, is loaded sequentially. Sequential loading ensures continuity within the MRI dataset and preserves spatial relationships between slices, thereby maintaining the 3D anatomical structure of the heart. Subsequently, each 2D slice is converted to a grayscale format to emphasize intensity variations across tissue types. Converting to Grayscale format eliminates color information that is irrelevant for medical imaging, instead focusing on intensity values that correspond to tissues such as myocardium, blood, and fat. Later, a two-dimensional Gaussian Blur filter is applied to each 2D slice to reduce noise and smoothen the images. The filtering step minimizes high-frequency noise that could interfere with accurate segmentation, particularly in detailed images, and enhances the definition of cardiac structures for subsequent processing stages. Then, thresholding is performed on each 2D slice to isolate the heart region based on the intensity values of cardiac tissues. Threshold values are selected to differentiate heart tissues from surrounding structures, wherein the thresholding operation is conducted independently for each 2D slice, ensuring accurate identification of cardiac structures within each frame. Next, a plurality of filtered 2D slices is generated for each 2D slice after thresholding by retaining only the pixels corresponding to the heart region, wherein the filtering removes extraneous tissues, ensuring that only the relevant cardiac tissue is preserved for further analysis. Finally, each among the plurality of filtered 2D slices are combined to generate a 3D mask that isolates the heart region from surrounding tissues across the entire MRI volume. The subtraction of the plurality of filtered 2D slices from the original 3D volume emphasizes the 3D cardiac structure.

Further, the method via the one or more hardware processors select a region of interest (ROI) from the 3D cardiac structure generated after segmentation of the plurality of multi-modal images. The ROI is defined based on the specific area of the heart being studied, such as the ventricles, atria, or heart valves. The ROI can be of any shape and size, allowing flexibility depending on the region under analysis. The objective of selecting the ROI is analyzing the blood flow in certain cardiac regions, such as through a valve or a specific vessel. Further, the method via the one or more hardware processors is configured to concurrently estimate a reference blood flow velocity from the 3D cardiac structure, especially within the ROI. The reference blood flow velocity estimation includes the following steps: calculating a set of flow vectors within the ROI using an optical flow algorithm such as Fernback optical flow algorithm. The set of flow vectors are calculated from speed of the blood through the ROI. The optical flow algorithm works by analyzing pixel intensity changes between successive MRI frames (slices) over time. In 3D volumes, analyzing pixel intensity means that for each frame (slice) of the MRI, the optical flow algorithm calculates an optical flow (motion) of the blood from one frame/slice to the next, capturing both spatial and temporal variations. The optical flow over the 3D cardiac structure is then scaled using blood flow information at specific regions (e.g. through Mitral valve, aortic valve etc.) obtained from the plurality of multi-modal images, say a 2D Echo report. This scaled 3D blood flow is termed as reference blood flow. The speed of the blood flow calculated from the above formulae in cm/s is considered as the Reference blood flow velocity.

Further, the method via the one or more hardware processors is configured to generate a patient specific reference model by registering the reference blood flow over the 3D cardiac structure. Then, the method via the one or more hardware processors measure a blood flow velocity profile and a pressure profile throughout the ROI using modified Lattice Boltzmann Method based computation model (LBM model). The blood flow velocity profile is computed by performing a sequence of steps, wherein the sequence of steps include: Initially, computing wall velocity and pressure profile of blood flow within the ROI based on structural analysis of the 3D cardiac structure. The wall velocity is imported from the MRI 3D structural analysis. And, concurrently obtaining a set of boundary conditions for the ROI, wherein the set of boundary conditions includes an inlet velocity, and an inlet pressure of the blood flow captured from a lumped zero-dimensional (0D) hemodynamic model. The lumped 0D hemodynamic model is a closed-loop, real-time, cardiovascular simulation model representing dynamics of four cardiac chambers, heart valves, and lumped pulmonary and systemic circulation. Finally, calculating the blood flow velocity profile and pressure profile within the ROI based on the wall velocity, pressure profile and the set of boundary conditions using the modified LBM model.

Further, the method via the one or more hardware processors is configured to generate a four-dimensional (4D) cardiac model by registering the blood flow velocity profile and the pressure profile with the 3D cardiac structure using optical flow algorithms. The 4D cardiac model also embeds a volume derived from the blood flow velocity and the pressure profile as its fourth dimension. Finally, the 4D cardiac model is calibrated using the reference model, wherein the blood flow velocity profile is calibrated with the reference blood flow velocity and wherein the calibration is made by adjusting the inlet velocity and inlet pressure captured from the lumped 0D hemodynamics model.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system generating a personalized four-dimensional (4D) cardiac model by registering hemodynamics over a three-dimensional (3D) cardiac structural model, according to some embodiments of the present disclosure.

FIG. 2 is a flow diagram illustrating a method for generating the personalized 4D cardiac model by registering hemodynamics the over the 3D cardiac structural model in accordance with some embodiments of the present disclosure.

FIG. 3 depicts an overview of step-by-step procedure for generating a personalized 4D cardiac model, according to some embodiments of the present disclosure.

FIG. 4A through FIG. 4B illustrates geometries for modelling of blood flow across two ventricles within a heart, according to some embodiments of the present disclosure. The blood flow velocity is changed when oxygenated blood and deoxygenated blood mixes due to defects in interventricular septum.

FIG. 5A through 5D depicts blood flow velocity profiles, wherein FIG. 5A and 5B represents unsteady state and FIG. 5C and 5D represents steady state for counter blood flow configurations, according to some embodiments of the present disclosure. Compartment at the right has higher pressure allowing blood flow to the left through the connecting tube representing intraventricular septal defect. FIG. 5B and FIG. 5D represent the velocity profile on the yz-plane at the middle of the left compartment.

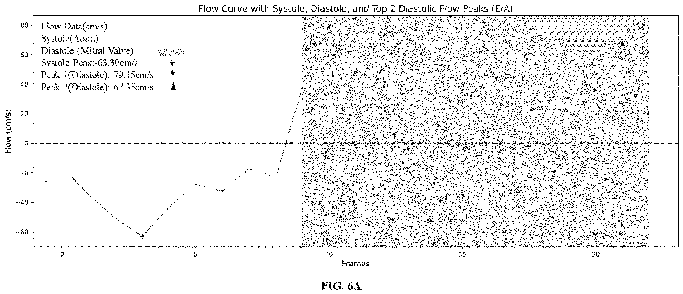

FIG. 6A depicts reference blood flow velocity, according to some embodiments of the present disclosure. The white region is Systole part where the heart contracts, and blood is ejected from the left ventricle through the aortic valve into the aorta. The grey region is Diastole where Mitral valve opens allowing oxygenated blood from the left atrium to flow into the left ventricle. In the legend the peak velocities are given in cm/s. In Systole from the left ventricle the blood comes out hence the velocity is treated as negative and in Diastole the blood flows into the left ventricle hence velocity is treated as positive.

FIG. 6B depicts the reference blood flow velocity and the blood flow velocity profile from optical flow algorithm , according to some embodiments of the present disclosure.. The dotted lines are referenced flow (obtained by finding the scaling factor). The white region is systole and the grey region is diastole.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

## GLOSSARY:

The term "4D model" refers to an integrated cardiac structural-functional model, where three-dimensional flow functionality and its changes with beating of heart is embedded over the three-dimensional cardiac structure

The term "hemodynamics" refers to zero-dimensional blood flow dynamics, capturing pressure and volumetric changes responsible for blood flow.

The term "functional model" refers to three-dimensional blood flow model, which is a combination of zero-dimensional lumped model and three-dimensional flow model.

The term "multi-modal images" refers to images of heart from different modalities like ECHO, MRI, CT etc.

Conventionally three-dimensional (3D) structural models for representing the cardiac structure are used for studying cardiac functions and monitoring cardiac anomalies. These 3D structural models are mostly static and provide no functional information or personalization to individual apart from structural information from imaging modalities like magnetic resonance imaging (MRI) and computed tomography (CT). Few works attempted building a 3D dynamic model of the heart but was limited to only a part of the heart and not the entire heart. Such a partial model is not useful to generate a digital twin of a beating heart. Further, registration of a hemodynamic model, which captures flow of blood for a given subject, on the 3D structural model is not straightforward given the complexity of real heart model and 3D representation of physical heart. Also, some of the existing technique uses "interventional imaging system" such as Transesophageal echocardiogram (TEE) or catheters which are time consuming and not feasible for practical cases, while some methods have tried registering hemodynamic model on adult heart, dealing with pediatric heart is even more complex and sensitive.

Few works strived in fabricating 4D models, embeds time as the 4th dimension over structural information. However, these 4D models were not capable of embedding the blood flow dynamics or hemodynamics with the cardiac structure and hence reconstructing the structure- function interplay for a person's heart is not realized in these models.

To overcome the above-mentioned drawbacks of existing methods, embodiments of the present disclosure provide a method and system for generating a personalized four-dimensional (4D) cardiac model by registering hemodynamics over a three-dimensional (3D) cardiac structural model. The 4th dimension embedded in the generated personalized 4D cardiac model is the blood flow dynamics of the heart. Flow dynamics reflects pressure volume variation in the cardiac structure for normal and pathological conditions in sync with cardiac cycles allowing better understanding and visualization of the structure-function interplay. Thus, the personalized 4D cardiac model proposed herein represents a beating heart which helps surgeons in surgery pre-planning and simulating different abnormal conditions.

4D cardiac modelling disclosed herein is an effective technique to aid in pre-surgical planning and simulation of disease condition to study the effect on various cardiac parameters. Surgeons can use the personalized 4D cardiac model, also referred to as 4D cardiac model herein after, to plan their surgery, especially for pediatric cases where the cardiac structure is too small, and operation time is very critical. Enhanced and simultaneous rendering of multiple information during cardiac surgical procedures via the proposed 4Dcardiac model along with simulated hemodynamic flow, beforehand knowledge of probable arrhythmic source can reduce surgical time and improve surgical outcomes.

The method proposed first generates a personalized 3D structural model or a personalized digital twin of a heart of a subject under observation and then applies the hemodynamics of the subject to the personalized 3D structural model.

The generation of the 3D structural model for the subject includes the following process. Firstly, a plurality of multi-modal cardiac images such as computed tomography (CT)/ magnetic resonance imaging (MRI) are received. Segmentation process is performed on the received plurality of multi-modal cardiac images by implementing a 3D GAN [Generative Adversarial Network] that includes 3D contextual information to segment and identify a plurality of cardiac substructures. Segmentation and identification of various substructures of the heart at different cardiac phases of end-systole and end-diastole also helps in the extraction of ventricular function information such as stroke volume, ejection fraction, myocardium thickness, etc., which are essential in designing a 3D heart. With the implementation of the 3D GAN that includes 3D contextual information, segmentation, and identification of the substructures at different cardiac phases are done more accurately. A 3D solid model of the heart is generated based on the person specific ventricular function information after segmentation. Further, the personalized 3D cardiac structural model is designed by incorporating volume and domain-related physiological features from auricular and ventricular function information extracted from the plurality of cardiac substructures.

Further, from the personalized 3D cardiac structural model or 3D cardiac structure of the heart as well as data from echo or doppler, a functional model of the person specific heart is built. The functional model includes a hemodynamic model, and a bio-mechanics model. The hemodynamic model, responsible for generating blood flow dynamics across cardiac structure along systemic and pulmonary vasculature, is generated from a 3D volumetric mesh created from the personalized 3D cardiac structure. The hemodynamics model itself comprises of lumped model hemodynamics that replicates systemic and pulmonary circulation and a 3D computational fluid dynamics model that computes cardiac bio-mechanical features. All the functional blocks are interlinked with one another.

The present disclosure provides a method for generating a personalized four-dimensional (4D) cardiac model by overlaying the hemodynamics flow on the 3D structural model unlike the prior art which overlays electrophysiology over the structure.

The major contributions of the proposed system are automatic recognition of anatomical segments from cardiac MRI, able to provide personalized functional model of the patient's heart and overlay the functional model on top of the personalized 3D structural model and thus providing surgeons with an immersive display to perform digital planning and digital "what-if" simulations on the personalized digital twin on the patient's heart.

Quantifying and registering blood flow in 3D cardiac MRI is a critical step in understanding and diagnosing cardiovascular conditions. Accurate analysis of blood flow patterns provides essential insights into cardiac function, helping clinicians assess heart health, detect abnormalities, and plan appropriate treatments. However, analyzing 3D MRI data is complex due to the volume of information, requiring precise flow measurements over time while considering the heart's 3D anatomical structure.

The proposed methodology leverages advanced image processing techniques designed to address the above-mentioned challenges. The process begins with the extraction of the heart region from the 3D MRI data using histogram-based image segmentation. At this step, the heart region is isolated from surrounding tissues and a 3D cardiac structure is, ensuring that the subsequent flow analysis focuses solely on the relevant structures. Once the heart region is segmented, a Region of Interest (ROI) is selected, wherein the ROI varies in shape and size depending on the specific cardiac region under study. Focusing on a well-defined ROI improves processing efficiency, enabling faster analysis by narrowing down the area of interest. For blood flow quantification, the Fernback optical flow algorithm is applied to analyze pixel intensity changes across successive MRI slices. The optical flow technique tracks blood flow through the heart, providing a time-resolved, volumetric view of the blood flow dynamics across the 3D volume. The final step involves registering the flow data across the 3D MRI volume, aligning it spatially to ensure accurate quantification and facilitate a comprehensive understanding of the flow patterns throughout the entire heart. This approach enables efficient and precise quantification of cardiac blood flow in 3D MRI, offering critical information for clinical decision-making and advancing cardiovascular research.

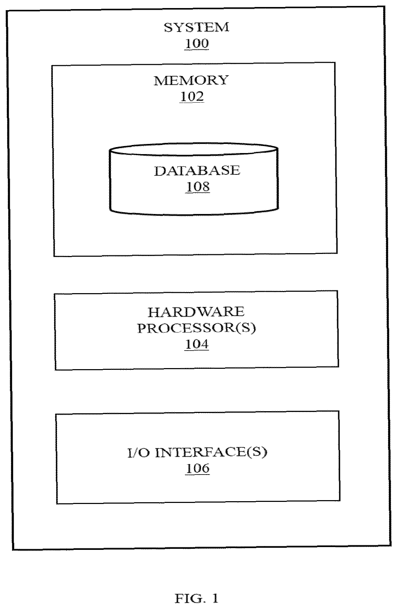

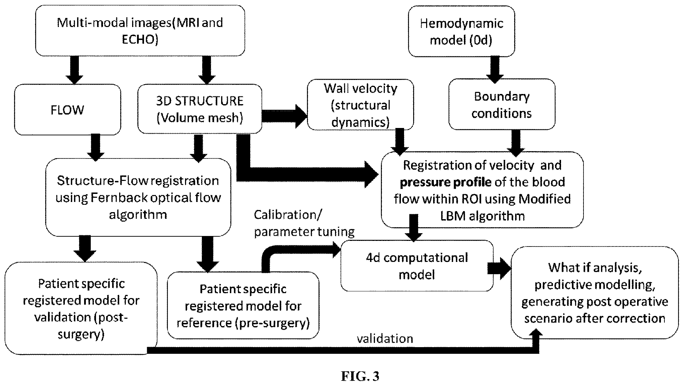

Referring now to the drawings, and more particularly to FIG. 1 through FIG. 6B, where similar reference characters denote corresponding features consistently throughout the figures, there are shown preferred embodiments and these embodiments are described in the context of the following exemplary system and/or method.

FIG. 1 illustrates an exemplary block diagram of a system 100 generating the personalized 4D cardiac model by registering hemodynamics over a personalized 3D cardiac structural model, according to some embodiments of the present disclosure. In an embodiment, the system 100 includes one or more processors 104, communication interface device(s) 106 or Input/Output (I/O) interface(s) 106 or user interface 106, and one or more data storage devices or memory 102 operatively coupled to the one or more processors 104. The memory 102 comprises a database 108. The one or more processors 104 that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) 106 receives a material characteristic data and a user query as input and gives recommendation of matching materials as output.

The memory 102 may include any computer-readable medium known in the art including, for example, volatile memory, such as static random-access memory (SRAM) and dynamic random- access memory (DRAM), and/or non-volatile memory, such as read only memory (ROM), erasable programmable ROM, flash memories, hard disks, optical disks, and magnetic tapes.

In an embodiment, the memory 102 includes a plurality of modules 110 for generation of 3D cardiac structural model and 4D cardiac model. The plurality of modules 110 include programs or coded instructions that supplement applications or functions performed by the system 100 for executing one or more steps involved in the process of generating the 4D cardiac model. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

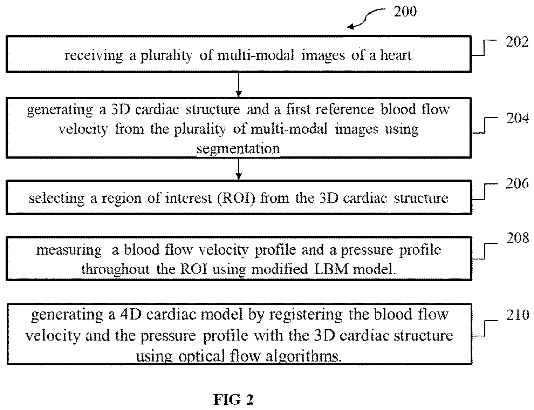

Functions of the components of system 100 are explained in conjunction with flow diagram depicted in FIG. 2 for generating the personalized four-dimensional (4D) cardiac model.

In an embodiment, the system 100 comprises one or more data storage devices or the memory 102 operatively coupled to the processor(s) 104 and is configured to store instructions for execution of steps of the method (200) depicted in FIG. 2 by the processor(s) or one or more hardware processors 104. The steps of the method of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practically. Further, some steps may be performed simultaneously.

FIG. 2 is a flow diagram illustrating method 200 of generating a personalized 4D cardiac model of heart by registering a hemodynamics flow over a personalized 3D cardiac structural model, according to some embodiments of the present disclosure. The hemodynamics flow includes a blood flow velocity and a pressure profile within a selected region of the heart under study.

Now, referring to FIG. 2, at step 202 of the method 200, the one or more hardware processors is configured to receive a plurality of multi-modal images of the heart. The plurality of multi-modal images includes but not limited to Magnetic Resonance Imaging (MRI), and echocardiography (ECHO) images of the heart. Then, at step 204 of the method 200, the one or more hardware processors are configured to generate a three-dimensional (3D) cardiac structure from the plurality of multi-modal images. The 3D cardiac structure is generated utilizing a histogram-based image segmentation approach. At this sept the heart region is isolated from surrounding tissues to ensure that subsequent flow analysis is focused solely on the relevant anatomical structures. The extraction of the heart region is a critical step in the in the method(200), as it ensures that the flow analysis remains accurate and is restricted to the appropriate cardiac structures. By eliminating surrounding tissues such as muscles, lungs, and ribs, the process facilitates precise identification of the heart region within the plurality of the multi-modal images. The extraction of the heart region and generation of the 3D cardiac structure involves the following steps:

Initially, the plurality of multi-modal images, for example MRI dataset comprising a series of two-dimensional (2D) slice frames forming 3D volume, is loaded sequentially. The sequential loading ensures continuity within the MRI dataset and preserves spatial relationships between slices, thereby maintaining the 3D anatomical structure of the heart. Subsequently, each 2D slice is converted to a grayscale format to emphasize intensity variations across tissue types. Converting to Grayscale format eliminates color information that is irrelevant for medical imaging, instead focusing on intensity values that correspond to tissues such as myocardium, blood, and fat.

Later, a two-dimensional Gaussian Blur filter is applied to each 2D slice to reduce noise and smoothen the images. The filtering step minimizes high-frequency noise that could interfere with accurate segmentation, particularly in detailed images, and enhances the definition of cardiac structures for subsequent processing stages. Then, thresholding is performed on each 2D slice to isolate the heart region based on the intensity values of cardiac tissues. Threshold values are selected to differentiate heart tissues from surrounding structures, wherein the thresholding operation is conducted independently for each 2D slice, ensuring accurate identification of cardiac structures within each frame. Next, a plurality of filtered 2D slices is generated for each 2D slice after thresholding by retaining only the pixels corresponding to the heart region, wherein the filtering removes extraneous tissues, ensuring that only the relevant cardiac tissue is preserved for further analysis. Finally, each among the plurality of filtered 2D slices are combined to generate a 3D mask that isolates the heart region from surrounding tissues across the entire MRI volume. The subtraction of the plurality of filtered 2D slices from the original 3D volume emphasizes the 3D cardiac structure.

Now at step 206 of the method 200, the one or more hardware processors are configured to select a region of interest (ROI) from the 3D cardiac structure generated after segmentation of the plurality of multi-modal images. The ROI is defined based on the specific area of the heart being studied, such as the ventricles, atria, or heart valves. The ROI can be of any shape and size, allowing flexibility depending on the region under analysis. The objective of selecting the ROI is analyzing the blood flow in certain cardiac regions, such as through a valve or a specific vessel. Defining a smaller ROI not only ensures the focus on relevant areas, but it also significantly enhances processing speed, as the size of the ROI is smaller compared to the original 3D MRI volume. Also, at the same step, a reference blood flow velocity is estimated from the 3D cardiac structure, especially within the ROI.

The reference blood flow velocity estimation includes the following steps: calculating a set of flow vectors within the ROI using an optical flow algorithm such as Fernback optical flow algorithm. The set of flow vectors are calculated from speed of the blood through the ROI. The optical flow algorithm works by analyzing pixel intensity changes between successive MRI frames (slices) over time. In 3D volumes, analyzing pixel intensity means that for each frame (slice) of the MRI, the optical flow algorithm calculates an optical flow (motion) of the blood from one frame/slice to the next, capturing both spatial and temporal variations.

The Fernback optical flow algorithm is based on the assumption that the intensity of blood (any moving object in general) changes between consecutive MRI frames/slices. The Fernback optical flow algorithm computes the displacement of pixel intensities across space and time. Since (blood)flow calculations are based on the assumption of pixel displacements, conversion of the blood flow to a real-world unit is necessary and hence conversion requires a Speed factor. The speed factor is accountable for the relationship between motion of pixels and actual physical motion in the real world. The speed factor is essential because the flow field is generally tied to the resolution of imaging sensors, say a camera and distance of the object from the camera. Hence optical flow cannot be translated into a quantitative measure of the true velocity of the objects in the scene.

Optical Flow Calculation: Optical blood flow is the speed of blood flow through the ROI obtained from the plurality of multi-modal images, MRI here. The optical blood flow is computed frame by frame from the plurality of multi-modal images, providing blood flow velocities in pixels per second.

Maximum Flow Frame Identification: The frame with the maximum optical flow is identified, typically corresponding to peak flow for systole and diastole from different views.

Echo Data for Calibration: Peak velocities from echo data (systole and diastole) provides ground truth for actual flow speeds in physical units (e.g., cm per second).

Scale Factor Calculation: A scale factor is calculated by dividing the actual peak velocity from the echo data by the maximum optical flow velocity for each phase:

$$speed\ factor\ =\ \frac{Peak\ velocity\ from\ echo}{Max\ flow\ from\ optical\ flow}$$

Frame Speed Adjustment: The scale factor is applied to adjust all frame velocities from the optical flow data to real-world units, ensuring accurate flow speed measurements for both systole and diastole.

Since the flow velocities during systole and diastole are generally different, separate scale factors are used for each phase. This approach accounts for the physiological differences in flow characteristics and ensures that the calibration process is phase-specific, providing more accurate velocity estimates for both the systolic and diastolic phases of cardiac cycle.

Mathematically, the optical flow can be described as follows: Suppose an object with intensity $I(x,y,t)$ So, the new intensity would be $I(x + dx, y + dy, t + dt)$ So, if the pixel intensities are constant in each frame, then by Taylor approximation -

$$\frac{dI}{dx}\partial x\ +\ \frac{dI}{dy}\partial y\ +\ \frac{dI}{dt}\partial t\ =\ 0$$

Now by dividing with

$$\frac{dI}{dx}f_x\ +\ \frac{dI}{dy}f_v\ +\ \frac{dI}{dt}\ =\ 0$$

Where, $f_x\ =\ \frac{dx}{dt}$ and $f_y\ =\ \frac{dy}{dt}$. Here, $\frac{dI}{dx}, \frac{dI}{dy}$ and $\frac{dI}{dt}$ are the horizontal, vertical and along the time respectively.

Now, having $x, y, f_x, f_y$ create grid points with spacing determined by the step argument. The grid points are used to calculate the flow vectors in the following points within the ROI.

The optical flow vectors at the grid points are extracted from the flow array. The magnitude of the flow vector is given by -

$$Magnitude\ =\ \sqrt{f_x^2\ +\ f_y^2}$$

The angle of each flow vector is computed using $\theta\ =\ \tan^{-1}\left(\frac{f_y}{f_x}\right)$, which gives the direction of the flow in radians.

Now calculating the speed of blood flow using the following formula -

$$speed\ =\ \frac{\left(\dfrac{\sum_{i=1}^{n}\sqrt{f_{x_i}^2\ +\ f_{y_i}^2}}{n}\ \cdot\ pc\right)}{dt}\cdot speed_{factor}$$

Where $pc$ is the pixel to centimeter scale which is extracted from the MRI dataset. $dt$ s the time gap between consecutive frames. $speed_{factor}$ the factor to scale the speed. The optical blood flow over the 3D cardiac structure is then scaled using blood flow information at specific regions (e.g. through Mitral valve, aortic valve etc.) obtained from the plurality of multi-modal images, say a 2D Echo report. This scaled 3D blood flow is termed as reference blood flow. The speed of the blood flow calculated from the above formulae in cm/s is considered as the Reference blood flow velocity.

Now, at the same step a patient specific reference model is generated by registering the reference blood flow over the 3D cardiac structure. At step 208 of the method (200) the one or more hardware processors are configured for measuring a blood flow velocity profile and a pressure profile throughout the ROI using modified Lattice Boltzmann Method based computation model (LBM model). In the development of the computational fluid flow framework, LBM model is implemented on a simplified geometry of two parallel cylinders representing left and right compartments with a connecting tube representing septal defect. Blood flow through the connecting tube leads to the undesired mixing of oxygenated and deoxygenated blood in two compartments, which mimics the mixing of blood due to defect in interventricular septum. This geometry is further converted to a surface mesh of triangular elements and submerged in the Eulerian structured mesh of lattice points. For measuring the blood velocity profile, wherein the blood flow velocity profile is computed by performing a sequence of steps, wherein the sequence of steps include: Initially, computing wall velocity and pressure profile of blood flow within the ROI based on structural analysis of the 3D cardiac structure. The wall velocity is imported from the MRI 3D structural analysis. And, concurrently obtaining a set of boundary conditions for the ROI, wherein the set of boundary conditions includes an inlet velocity and an inlet pressure of the blood flow captured from a lumped zero-dimensional (0D) hemodynamic model. The lumped 0D hemodynamic model is a closed-loop, real-time, cardiovascular simulation model representing dynamics of four cardiac chambers, heart valves, and lumped pulmonary and systemic circulation. Finally, calculating the blood flow velocity profile and pressure profile within the ROI based on the wall velocity, pressure profile and the set of boundary conditions using the modified LBM model.

Further, at step 210 of the method 200, the one or more hardware processors are configured for generating a four-dimensional (4D) cardiac model by registering the blood flow velocity profile and the pressure profile with the 3D cardiac structure using optical flow algorithms. The 4D cardiac model also embeds a volume derived from the blood flow velocity and the pressure profile as its fourth dimension. Finally, the 4D cardiac model is calibrated using the reference model, wherein the blood flow velocity profile is calibrated with the reference blood flow velocity and wherein the calibration is made by adjusting the inlet velocity and inlet pressure captured from the lumped 0D hemodynamics model.

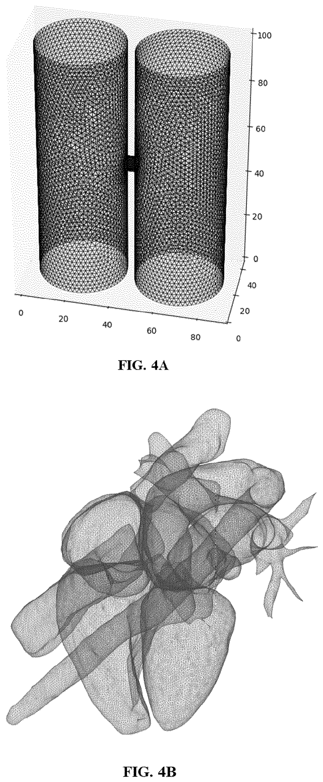

Now, referring to FIG. 4A through FIG. 4B, geometries for modelling of blood flow across two ventricles within a heart is illustrated, according to some embodiments of the present disclosure. The blood flow velocity is changed when oxygenated blood and deoxygenated blood mixes due to defects in interventricular septum.

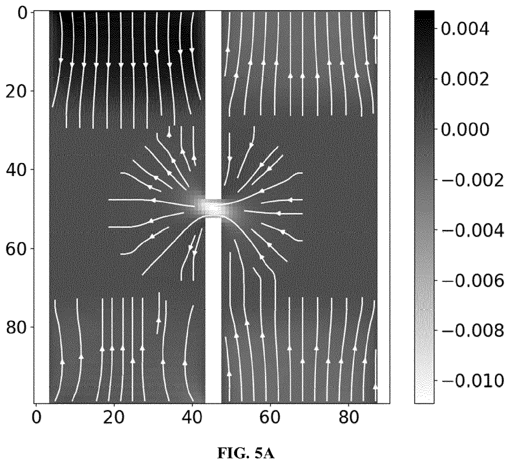

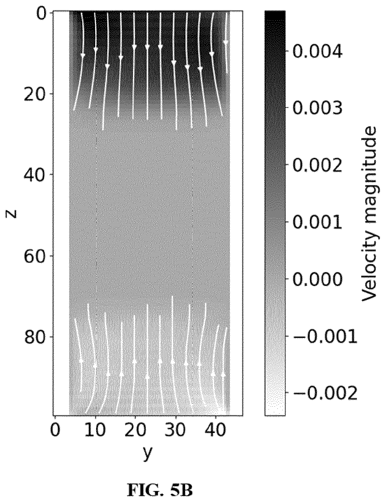

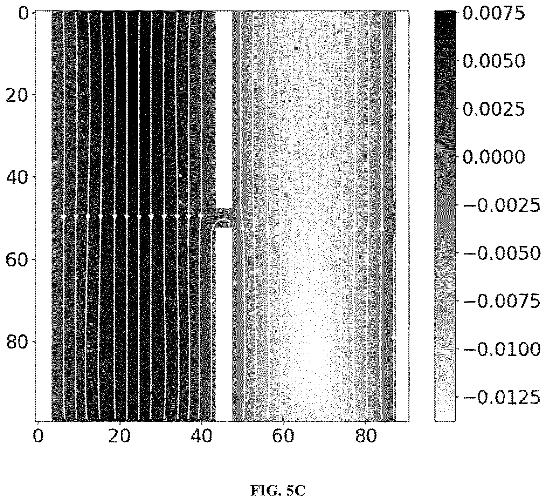

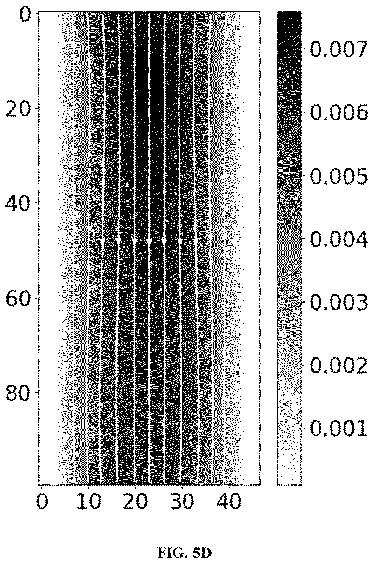

Referring to FIG. 5A through 5D, the blood flow velocity profiles are illustrated, wherein FIG. 5A and 5B represents unsteady state and FIG. 5C and 5D represents steady state for counter blood flow configurations, according to some embodiments of the present disclosure. Compartment at the right has higher pressure allowing blood flow to the left through the connecting tube representing intraventricular septal defect. FIG. 5B and FIG. 5D represent the velocity profile on the yz-plane at the middle of the left compartment.

Referring to FIG. 6A the reference blood flow velocity is depicted, according to some embodiments of the present disclosure. The white region is Systole part where the heart contracts, and blood is ejected from the left ventricle through the aortic valve into the aorta. The grey region is Diastole where Mitral valve opens allowing oxygenated blood from the left atrium to flow into the left ventricle. In the legend the peak velocities are given in cm/s. In Systole from the left ventricle the blood comes out hence the velocity is treated as negative and in Diastole the blood flows into the left ventricle hence velocity is treated as positive.

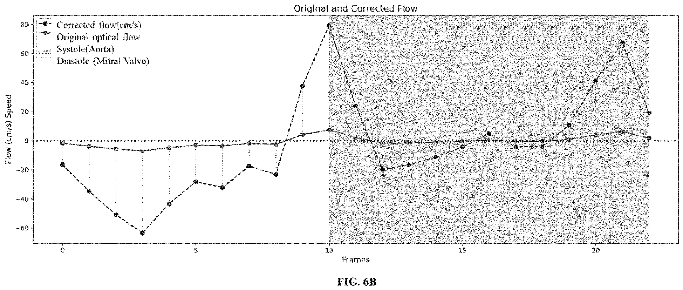

Referring to FIG. 6B the reference blood flow velocity and the blood flow velocity profile from optical flow algorithm is depicted, according to some embodiments of the present disclosure. The dotted lines are referenced flow (obtained by finding the scaling factor). The white region is systole and the grey region is diastole.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein addresses unresolved problem of generating a personalized four dimensional (4D) cardiac model by registering hemodynamics over a 3D cardiac structure. The embodiment, thus provides the 4D cardiac model that integrates blood flow velocity and pressure data, providing a comprehensive tool for pre- and post-surgical planning and analysis. Unlike existing techniques, which often rely on separate assessments of cardiac anatomy and hemodynamics, using invasive methods, the present disclosure provides a unified model that captures the person specific dynamic interaction between cardiac structures and blood flow throughout the cardiac cycle. Further, the 4D cardiac model enables clinicians to visualize and quantify complex flow patterns and pressure distributions within the region of interest in the heart, facilitating more precise surgical planning and outcome prediction especially in pediatric surgical planning.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200) for generating a four-dimensional (4D) cardiac model, the method comprising:

   receiving(202), via one or more hardware processors, a plurality of multi-modal images of a heart of a subject under observation, wherein the plurality of multi-modal images includes Magnetic Resonance Imaging (MRI) and echocardiography (ECHO) images;
   generating(204), via the one or more hardware processors, a three-dimensional (3D) cardiac structure from the plurality of multi-modal images using segmentation;
   selecting (206), via the one or more hardware processors, a region of interest (ROI) and estimating a reference blood flow velocity from the 3D cardiac structure;
   measuring (208), via the one or more hardware processors, a blood flow velocity profile and a pressure profile throughout the ROI using modified Lattice Boltzmann Method based computation model (LBM model); and
   generating (210), via the one or more hardware processors, a four-dimensional (4D) cardiac model by registering the blood flow velocity profile and the pressure profile with the 3D cardiac structure using optical flow algorithms, wherein the 4D cardiac model is calibrated using the 3D cardiac structure, and the reference blood flow.

2. The method as claimed in claim 1, wherein obtaining the reference blood flow velocity from the 3D cardiac structure comprises:

obtaining an optical flow of blood within the ROI by analyzing pixel intensities;

obtaining a flow velocity per pixel based on the optical flow;

identifying a peak flow velocity from the flow velocity per pixel values;

estimating a plurality of scale factors corresponding to systole and diastole phases of the ROI from the peak flow velocity and an actual peak;

applying the scale factors to calibrate the flow velocity from the optical flow;

calculating a set of flow vectors corresponding to the calibrated flow velocity within the ROI;

estimating magnitude and angle of each of the set of flow vectors;

calculating speed of blood flow within the ROI based on the magnitude and angle of the set of flow vectors;

correcting/calibrating the speed of blood flow using an actual speed of blood flow obtained from the plurality of multi-modal images; and

estimating the reference blood flow velocity based on the corrected speed of blood flow within the ROI.

3. The method as claimed in claim 2, wherein the plurality of scale factors is obtained based on systolic and diastolic phases of the ROI, and wherein the actual peak velocity is obtained from the plurality of multi-modal images.

4. The method as claimed in claim 1, wherein computing the blood flow velocity profile comprises:

computing wall velocity and pressure profile of blood flow within the ROI based on structural analysis of the 3D cardiac structure;

concurrently obtaining a set of boundary conditions for the ROI;

wherein the set of boundary conditions includes an inlet velocity, and an inlet pressure of the blood flow captured from a lumped zero-dimensional (0D) hemodynamic model; and

wherein the lumped 0D hemodynamic model is a closed-loop, real-time, cardiovascular simulation model representing dynamics of four cardiac chambers, heart valves, and lumped pulmonary and systemic circulation; and

calculating the blood flow velocity profile and pressure profile within the ROI based on the wall velocity, and the set of boundary conditions using the modified LBM model.

5. The method as claimed in claim 1, wherein the 4D cardiac model provides a personalized functional and structural model of the heart.

6. A system (100), comprising:

a memory (102) storing instructions;

one or more communication interfaces (106); and

one or more hardware processors (104) coupled to the memory (102) via the one or more communication interfaces (106), wherein the one or more hardware processors (104) are configured by the instructions to:

receive a plurality of multi-modal images of a heart, wherein the plurality of multi-modal images includes Magnetic Resonance Imaging (MRI) and echocardiography (ECHO) images;

generate a three-dimensional (3D) cardiac structure from the plurality of multi-modal images using segmentation;

selecting a region of interest (ROI) and estimating a reference blood flow velocity from the 3D cardiac structure;

measure a blood flow velocity profile and a pressure profile throughout the ROI using modified Lattice Boltzmann Method based computation model (LBM model); and

generate a four-dimensional (4D) cardiac model by registering the blood flow velocity and the pressure profile with the 3D cardiac structure using optical flow algorithms, wherein the 4D cardiac model is calibrated using the 3D cardiac structure, and the reference blood flow.

7. The system as claimed in claim 6, wherein the one or more hardware processors are configured to obtain the reference blood flow velocity is from the 3D cardiac structure by performing the following steps:

obtaining an optical flow of blood within the ROI by analyzing pixel intensities,

obtaining a flow velocity per pixel based on the optical flow;

identifying a peak flow velocity from the flow velocity per pixel values;

estimating a plurality of scale factors corresponding to systole and diastole phases of the ROI from the peak flow velocity and an actual peak;

applying the scale factors to calibrate the flow velocity from the optical flow;

calculating a set of flow vectors corresponding to the calibrated flow velocity within the ROI;

estimating magnitude and angle of each of the set of flow vectors;

calculating speed of blood flow within the ROI based on the magnitude and angle of the set of flow vectors;

correcting/calibrating the speed of blood flow using an actual speed of blood flow obtained from the plurality of multi-modal images; and

estimating the first reference blood velocity based on the corrected speed of blood flow within the ROI.

8. The system as claimed in claim 7, wherein one or more hardware processors are configured to obtain the plurality of scale factors based on systolic and diastolic phases of the ROI and wherein the actual peak velocity is obtained from the plurality of multi-modal images.

9. The system as claimed in claim 6, wherein one or more hardware processors are configured to compute the blood flow velocity profile by performing:

computing wall velocity and pressure profile of blood flow within the ROI based on structural analysis of the 3D cardiac structure;

concurrently obtaining a set of boundary conditions for the ROI,

wherein the set of boundary conditions includes an inlet velocity and an inlet pressure of the blood flow captured from a lumped zero-dimensional (0D) hemodynamic model; and

wherein the lumped 0D hemodynamic model is a closed-loop, real-time, cardiovascular simulation model representing dynamics of four cardiac chambers, heart valves, and lumped pulmonary and systemic circulation.

calculating the blood flow velocity profile and pressure profile within the ROI based on the wall velocity, and the set of boundary conditions using the modified LBM model.

10. The system as claimed in claim 6, wherein the one or more hardware processors are configured to calibrate the 4D cardiac model using the 3D cardiac structure, the reference blood flow and the blood flow velocity profile, and wherein the 4D cardiac model provides a personalized functional and structural model of the heart.

11. One or more non-transitory machine-readable information storage mediums comprising one or more instructions which when executed by one or more hardware processors cause:

receiving a plurality of multi-modal images of a heart, wherein the plurality of multi-modal images includes Magnetic Resonance Imaging (MRI) and echocardiography (ECHO) images;

generating a three-dimensional (3D) cardiac structure from the plurality of multi-modal images using segmentation;

selecting a region of interest (ROI) and estimating a reference blood flow velocity from the 3D cardiac structure;

measuring a blood flow velocity profile and a pressure profile throughout the ROI using modified Lattice Boltzmann Method based computation model (LBM model); and

generating a four-dimensional (4D) cardiac model by registering the blood flow velocity and the pressure profile with the 3D cardiac structure using optical flow algorithms, wherein the 4D cardiac model is calibrated using the 3D cardiac structure, and the reference blood flow.

12. The one or more non-transitory machine-readable information storage mediums of claim 11, wherein the reference blood flow velocity is obtained from the 3D cardiac structure by performing the following steps:

obtaining an optical flow of blood within the ROI by analyzing pixel intensities,

obtaining a flow velocity per pixel based on the optical flow,

identifying a peak flow velocity from the flow velocity per pixel values,

estimating a plurality of scale factors corresponding to systole and diastole phases of the ROI from the peak flow velocity and an actual peak;

applying the scale factors to calibrate the flow velocity from the optical flow;

calculating a set of flow vectors corresponding to the calibrated flow velocity within the ROI;

estimating magnitude and angle of each of the set of flow vectors;

calculating speed of blood flow within the ROI based on the magnitude and angle of the set of flow vectors;

correcting/calibrating the speed of blood flow using an actual speed of blood flow obtained from the plurality of multi-modal images; and

estimating the first reference blood velocity based on the corrected speed of blood flow within the ROI.

13. The one or more non-transitory machine-readable information storage mediums of claim 12, wherein the plurality of scale factors is obtained based on systolic and diastolic phases of the ROI and wherein the actual peak velocity is obtained from the plurality of multi-modal images.

14. The one or more non-transitory machine-readable information storage mediums of claim 11, wherein the blood flow velocity profile is computed by performing a sequence of steps, wherein the sequence of steps include:

computing wall velocity and pressure profile of blood flow within the ROI based on structural analysis of the 3D cardiac structure;

concurrently obtaining a set of boundary conditions for the ROI,

wherein the set of boundary conditions includes an inlet velocity and an inlet pressure of the blood flow captured from a lumped zero-dimensional (0D) hemodynamic model; and

wherein the lumped 0D hemodynamic model is a closed-loop, real-time, cardiovascular simulation model representing dynamics of four cardiac chambers, heart valves, and lumped pulmonary and systemic circulation.

calculating the blood flow velocity profile and pressure profile within the ROI based on the wall velocity, and the set of boundary conditions using the modified LBM model.

15. The one or more non-transitory machine-readable information storage mediums of claim 11, wherein the 4D cardiac model is calibrated using the 3D cardiac structure, the reference blood flow and the blood flow velocity profile and wherein the 4D cardiac model provides a personalized functional and structural model of the heart.

SYSTEM
100

MEMORY
102

DATABASE
108

HARDWARE
PROCESSOR(S)
104

I/O INTERFACE(S)
106

FIG. 1

200

receiving a plurality of multi-modal images of a heart — 202

generating a 3D cardiac structure and a first reference blood flow velocity from the plurality of multi-modal images using segmentation — 204

selecting a region of interest (ROI) from the 3D cardiac structure — 206

measuring a blood flow velocity profile and a pressure profile throughout the ROI using modified LBM model. — 208

generating a 4D cardiac model by registering the blood flow velocity and the pressure profile with the 3D cardiac structure using optical flow algorithms. — 210

**FIG 2**

```
┌─────────────────────────┐                              ┌─────────────────────┐
│ Multi-modal images(MRI and │                           │ Hemodynamic         │
│        ECHO)            │                               │ model (0d)          │
└─────────────────────────┘                              └─────────────────────┘
```

Multi-modal images(MRI and ECHO)

Hemodynamic model (0d)

FLOW

3D STRUCTURE (Volume mesh)

Wall velocity (structural dynamics)

Boundary conditions

Structure-Flow registration using Fernback optical flow algorithm

Registration of velocity and **pressure profile** of the blood flow within ROI using Modified LBM algorithm

Calibration/ parameter tuning

4d computational model

Patient specific registered model for validation (post-surgery)

Patient specific registered model for reference (pre-surgery)

What if analysis, predictive modelling, generating post operative scenario after correction

validation

FIG. 3

18

FIG. 4A

FIG. 4B

**FIG. 5A**

**FIG. 5B**

**FIG. 5C**

FIG. 5D

Flow Curve with Systole, Diastole, and Top 2 Diastolic Flow Peaks (E/A)

FIG. 6A

**FIG. 6B**

Europäisches
Patentamt

European
Patent Office

Office européen
des brevets

# EUROPEAN SEARCH REPORT

Application Number

EP 25 15 9273

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/228190 A1 (GEORGESCU BOGDAN [US] ET AL) 11 August 2016 (2016-08-11) * The whole document, in particular: Paragraphs [0020], [0022], [0032], [0039], [0044], [0072], [0075]; Figures 2, 3, 9, 10; Claims 1, 4. * | 1-15 | INV. G16H30/40 A61B5/00 A61B8/08 A61B8/00 G06T7/00 G16H50/50 A61B5/02 |
| X | SADEGHI REZA ET AL: "Impact of mixed valvular disease on coarctation hemodynamics using patient-specific lumped parameter and Lattice Boltzmann modeling", INTERNATIONAL JOURNAL OF MECHANICAL SCIENCES, PERGAMON PRESS, OXFORD, GB, vol. 217, 30 December 2021 (2021-12-30), XP086962953, ISSN: 0020-7403, DOI: 10.1016/J.IJMECSCI.2021.107038 [retrieved on 2021-12-30] * The whole document, in particular: *Abstract*, *1. Introduction* and *2. Material and method* on Pages 1 - 11; Figures 1 - 3. * | 1-15 | |
| A | WO 2020/048642 A1 (LIFEFLOW SP Z O O [PL]) 12 March 2020 (2020-03-12) * The whole document, in particular: Paragraph [059]. * | 1-15 | TECHNICAL FIELDS SEARCHED (IPC) G16H A61B G06T |

The present search report has been drawn up for all claims

| Place of search | Date of completion of the search | Examiner |
|---|---|---|
| Munich | 4 July 2025 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 9273

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2025

| Patent document cited in search report | Publication date | Patent family member(s) | | Publication date |
|---|---|---|---|---|
| US 2016228190 A1 | 11-08-2016 | NONE | | |
| WO 2020048642 A1 | 12-03-2020 | AU 2019335857 | A1 | 12-08-2021 |
| | | BR 112021013537 | A2 | 14-09-2021 |
| | | CA 3126313 | A1 | 12-03-2020 |
| | | CN 113365552 | A | 07-09-2021 |
| | | CN 118782255 | A | 15-10-2024 |
| | | EA 202191778 | A1 | 19-11-2021 |
| | | EP 3820357 | A1 | 19-05-2021 |
| | | EP 4122381 | A1 | 25-01-2023 |
| | | ES 2933276 | T3 | 03-02-2023 |
| | | ES 2963697 | T3 | 01-04-2024 |
| | | FI 3820357 | T3 | 15-12-2022 |
| | | HU E060191 | T2 | 28-02-2023 |
| | | HU E064142 | T2 | 28-02-2024 |
| | | IL 284704 | A | 31-08-2021 |
| | | JP 7241183 | B2 | 16-03-2023 |
| | | JP 2022517995 | A | 11-03-2022 |
| | | KR 20210117285 | A | 28-09-2021 |
| | | LT 3820357 | T | 25-10-2022 |
| | | PL 3820357 | T3 | 24-07-2023 |
| | | PL 4122381 | T3 | 05-02-2024 |
| | | PT 3820357 | T | 31-10-2022 |
| | | RS 64846 | B1 | 29-12-2023 |
| | | SA 521422498 | B1 | 30-04-2024 |
| | | SG 11202107506Q | A | 30-08-2021 |
| | | SI 3820357 | T1 | 31-03-2023 |
| | | US 2022093266 | A1 | 24-03-2022 |
| | | WO 2020048642 | A1 | 12-03-2020 |
| | | ZA 202105149 | B | 26-10-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421013196